(19) Europäisches Patentamt

European Patent Office

Office européen des brevets

(11) **EP 1 714 954 A1**

(12) **EUROPEAN PATENT APPLICATION**
published in accordance with Art. 158(3) EPC

(43) Date of publication:
**25.10.2006 Bulletin 2006/43**

(21) Application number: **05709894.9**

(22) Date of filing: **08.02.2005**

(51) Int Cl.:
*C07C 27/00* (2006.01)   *C07C 29/04* (2006.01)
*C07C 31/12* (2006.01)   *C07C 45/27* (2006.01)
*C07C 49/10* (2006.01)   *C07B 61/00* (2006.01)

(86) International application number:
**PCT/JP2005/001842**

(87) International publication number:
**WO 2005/075391 (18.08.2005 Gazette 2005/33)**

(84) Designated Contracting States:
**AT BE BG CH CY CZ DE DK EE ES FI FR GB GR HU IE IS IT LI LT LU MC NL PL PT RO SE SI SK TR**

(30) Priority: **10.02.2004 JP 2004033981**

(71) Applicant: **MARUZEN PETROCHEMICAL CO., LTD.**
**Tokyo 104-0032 (JP)**

(72) Inventors:
• **TSUNODA, Takashi**
  **7100845 (JP)**

• **AKAGISHI, Kenji**
  **Kawasaki-shi, Kanagawa 2110041 (JP)**
• **WATANABE, Atsushi**
  **7000071 (JP)**

(74) Representative: **Blake, John Henry Francis et al**
**Brookes Batchellor**
**102-108 Clerkenwell Road**
**London EC1M 5SA (GB)**

(54) **METHOD FOR PRODUCING ALCOHOL AND/OR KETONE**

(57)    A method for producing an alcohol and/or a ketone, wherein a raw material containing at least one alkene is reacted in the presence of steam under the contact with an oxide catalyst in a gas phase, to produce an alcohol and/or a ketone corresponding to said alkene(s), which comprises satisfying the following requirements: (a) the above oxide catalyst contains the oxide(s) of molybdenum and/or tin, (b) the above reaction is carried out under a condition wherein molecular oxygen is not fed and by the use of a system wherein said catalyst is circulated between a fluid bed reactor and a regenerator, and (c) a stripper is provided on the way from the regenerator to the reactor.

FIG.1

**Description**

TECHNICAL FIELD

The present invention relates to a method for producing an alcohol and/or a ketone from a corresponding alkene (s) using an oxide catalyst in the presence of steam in a gas phase.

BACKGROUND ART

Examples of methods for producing an alcohol and/or a ketone from a corresponding alkene(s) by a gas phase reaction in the presence of steam include production of acetone from propylene, production of methyl ethyl ketone (MEK) from 1-butene or 2-butene, production of cyclohexanone from cyclohexene, and production of tert-butanol from isobutene. Each of these products is industrially an extremely important chemical compound as a chemical starting material or a solvent.

Conventional techniques for the above-described reaction mainly include a Wacker-type reaction using a noble metal catalyst such as a palladium compound and a reaction using a composite oxide catalyst containing non-noble metals such as molybdenum, tungsten, tin, and cobalt.

Examples of the former Wacker-type reaction include the production of a carbonyl compound in the presence of olefin, oxygen, and steam using a catalyst in which palladium and/or a palladium compound and copper chloride are supported on a carrier such as silica or alumina (see e.g. Patent Document 1). Patent Document 1 describes, in the exemplary embodiment, the production of methyl ethyl ketone (MEK) from 1-butene using a catalyst in which palladium chloride and copper chloride are supported on silica.

In addition, examples using a chloride-free catalyst include production of acetaldehyde or a ketone by subjecting an olefin to gas-phase oxidation with oxygen or an oxygen-containing gas in the presence of steam using a catalyst having a palladium salt and a vanadyl salt supported on active carbon (see e.g. Patent Document 2). Patent Document 2 describes, in the exemplary embodiment, the production of acetone from propylene using a catalyst in which palladium sulfate and vanadyl sulfate are supported on active carbon.

However, these catalysts are obtained by the use of highly expensive noble metals, and further testing by the present inventors showed deteriorated activities of both catalysts in a short period of time.

On the other hand, examples of the latter reaction using a composite oxide catalyst containing non-noble metals include the reaction of olefin and oxygen in the presence of steam employing a catalyst consisting of a molybdenum oxide and a particulate tin oxide uniformly distributed on a carrier (see e.g. Patent Document 3). Patent Document 3 describes, in the exemplary embodiment, the production of acetone from propylene using a catalyst in which tin dioxide and molybdenum trioxide are supported on silica.

In addition, examples using similar catalysts include the reaction of a mixture of olefin and steam using a catalyst in which molybdenum oxide, tin oxide, and a specified amount of an alkali metal and/or an alkaline earth metal are supported on a carrier (see e.g. Patent Document 4). Patent Document 4 describes, in the exemplary embodiment, the production of MEK from trans-butene using a catalyst in which tin dioxide, molybdenum trioxide, and sodium are supported on silica.

Further, there is a method which involves, using a similar catalyst, alternately contacting the catalyst with a gas consisting of an olefin, steam and a small amount of oxygen as the reaction raw material and a gas containing a large amount of oxygen (see e.g. Patent Document 5). Patent Document 5 describes, in the exemplary embodiment, the production of MEK from n-butene using a catalyst in which tin dioxide and molybdenum trioxide are supported on silica.

However, reaction has been often conducted on a fixed bed in each of the above-described Patent Documents, and satisfactory results have not been obtained when the reaction is carried out using a system wherein a catalyst is circulated between a fluid bed reactor and a regenerator.

Patent Document 1: JP-A-49-72209
Patent Document 2: JP-A-59-163335
Patent Document 3: JP-B-47-8046
Patent Document 4: JP-A-49-61112
Patent Document 5: JP-B-49-34652

DISCLOSURE OF THE INVENTION

PROBLEMS TO BE SOLVED BY THE INVENTION

An object of the present invention is to provide a method for producing an alcohol and/or a ketone from a corresponding alkene(s) using an oxide catalyst in the presence of steam in a gas phase at an extremely high selectivity of the objective product while keeping the catalytic activity constant.

MEANS FOR SOLVING THE PROBLEMS

As the result of intensive studies for solving the above-described problems, the present inventors have found that, when (a) an oxide catalyst containing an oxide(s) of molybdenum and/or tin is used to (b) perform the above-described reaction which is carried out under a condition where molecular oxygen is not fed and by using a system wherein a catalyst is circulated between a fluid bed reactor and a regenerator, it can suit the purpose to (c) provide a stripper on the way from the regenerator to the fluid bed reactor. Based on this finding, the present invention is accomplished.

Thus, the invention relates to a producing process described below.

(1) A method for producing an alcohol and/or a ketone, wherein a raw material containing at least one alkene is contacted and reacted with an oxide catalyst in the presence of steam in a gas phase to produce an alcohol and/or a ketone corresponding to the alkene(s), which comprises satisfying the following requirements of (a) to (c):

(a) the above-described oxide catalyst contains an oxide(s) of molybdenum and/or tin;
(b) the above-described reaction is carried out under a condition where molecular oxygen is not fed and by the use of a system wherein the catalyst is circulated between a fluid bed reactor and a regenerator; and
(c) a stripper is provided on the way from the regenerator to the fluid bed reactor.

(2) The method described in term (1) wherein a stripper is further provided on the way from the reactor to the regenerator.
(3) The method described in term (1) or (2) wherein the alkene(s) is 1-butene and/or 2-butene.
(4) The method described in any of terms (1) to (3) wherein the atomic ratio X of molybdenum to the sum of tin and molybdenum contained in the above-described oxide catalyst ($(Mo/(Sn + Mo)$ where Mo is the number of molybdenum atoms in the oxide catalyst and Sn is the number of tin atoms in the oxide catalyst) is in the range of $0 \leq X < 0.50$.
(5) The method described in any of terms (1) to (3) wherein the atomic ratio X of molybdenum to the sum of tin and molybdenum contained in the above-described oxide catalyst ($(Mo/(Sn + Mo)$ where Mo is the number of molybdenum atoms in the oxide catalyst and Sn is the number of tin atoms in the oxide catalyst) is in the range of $0.01 \leq X \leq 0.24$.

EFFECT OF THE INVENTION

According to the invention, there can be provided a process for producing an alcohol and/or ketone, wherein the unnecessary loss of raw material is reduced and the selectivity of the objective product is increased.

BRIEF DESCRIPTION OF THE DRAWING

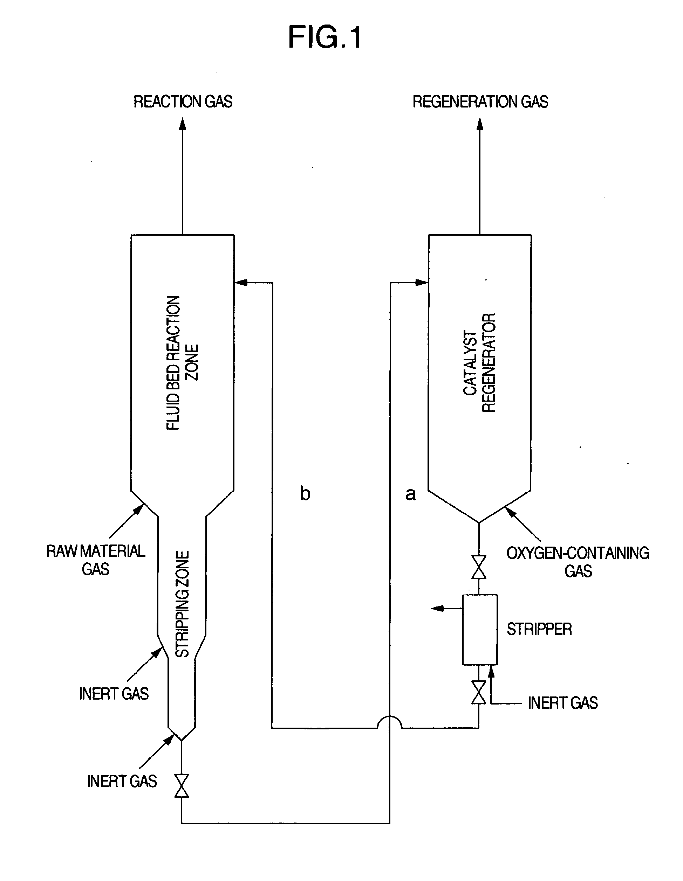

Fig. 1 is a schematic diagram consisting of a fluid bed reactor, a regenerator, and strippers.

DESCRIPTION OF SYMBOLS

a catalyst taking-out line
b catalyst recycling line

BEST MODE FOR CARRYING OUT THE INVENTION

The present invention is described below in detail.

The catalyst used in the process of the invention is a catalyst containing an oxide(s) of molybdenum and/or tin.

These oxides may be used alone, but are preferably employed as a mechanical mixture of both oxides of molybdenum and tin and/or a compound oxide thereof because there is the effect of improving the catalytic activity and the selectivity of the objective product. For further improving the catalytic activity and the selectivity of the objective product, an oxide of another element may be also added. The element is preferably an element belonging to the fourth, fifth, sixth, eighth, ninth, tenth, eleventh, fourteenth, or fifteenth group of the periodic table, and it is more preferably that the fourth group element is titanium or zirconium; the fifth group vanadium or niobium; the sixth group tungsten or chromium; the eighth group iron; the ninth group cobalt; the tenth group nickel; the eleventh group copper; the fourteenth group lead; and the fifteenth group bismuth, antimony, or phosphorus. The periodic table referred to herein is the 18-group type periodic table described in Chemical Society of Japan, "Kagaku Binran Kisohen I Fourth Revised Edition", Maruzen Co., Ltd. (1993), P. 1-56. A trace amount of an oxide of an alkali metal such as sodium, potassium, or rubidium or an alkaline earth metal such as magnesium, calcium, or barium may be further added.

Preferably, these oxides are also used by supporting on a suitable carrier. The carrier is preferably an inorganic oxide such as silica, silica-alumina, alumina, titania, silica-titania, zirconia or silica-zirconia; silica is particularly preferable. A clay such as kaolin or talc may be further added in order to increase the mechanical strength of catalyst.

When the oxide catalyst contains both oxides of molybdenum and tin, the atomic ratio X of molybdenum to the sum of tin and molybdenum ((Mo/(Sn + Mo) where Mo is the number of molybdenum atoms in the oxide catalyst and Sn is the number of tin atoms in the oxide catalyst) is preferably in the range of $0 \leq X < 0.50$ (X=0 means that no oxide of Mo is contained), more preferably $0.01 \leq X \leq 0.24$, even more preferably $0.05 \leq X \leq 0.24$, particularly $0.08 \leq X \leq 0.15$.

The atomic ratio X is 0 or more, but preferably more than 0 in terms of catalytic activity. In addition, it is preferably less than 0.50 to prevent the tendency of a molybdenum crystal to precipitate on the outside of catalyst during the calcination of the catalyst and to prevent the reduced fluidity of the catalyst.

A method for preparing the oxide catalyst used in the invention is described below in detail.

The catalyst preparation consists mainly of the steps of; 1) preparing a catalyst raw material solution and 2) drying the raw material solution and calcining a catalyst precursor.

1) The step of preparing a catalyst raw material solution

The chemical form of a raw material providing an oxide (hereinafter, the term "oxide" shall also include a compound oxide) which is the active species of catalyst is not particularly restricted. Preferably, a salt or a compound forming an oxide at 200 to 1,000°C is used. Examples thereof include nitrates, sulfates, acetates, oxalates, ammonium salts, chlorides, and hydroxides. In addition, a commercially available oxide may be used as it is.

Typically, one or more kinds of raw materials are well dissolved in water or a suitable solvent at 20 to 80°C. At this time, the pH of the solution may be adjusted to acidic or alkaline in order to increase the solubility of the raw material. In the case of poor solubility, hydrogen peroxide or the like may be added.

The raw material solution may be directly dried, but is preferably mixed well with a powder, a solution, a sol, or a gel containing a carrier component to support the oxide on a suitable carrier as described above.

At this time, when nitrates, sulfates, chlorides, and the like are used as raw materials for oxides, they are preferably converted to hydroxides by adding aqueous ammonia to avoid the generation of corrosive gas during a subsequent calcination step. To further control viscosity and the like, the pH of the mixture may be adjusted to acidic or alkaline.

2) The steps of drying the catalyst raw material solution and calcining a catalyst precursor

These steps consist of the step of drying to remove the solvent from the above-described catalyst raw material solution (hereinafter, the term "catalyst raw material solution" shall include that containing a carrier component) and to provide a catalyst precursor and the step of calcining the precursor to convert into an oxide catalyst.

A method for drying the catalyst raw material solution is not particularly restricted. Examples thereof include a method which involves removing the solvent from the catalyst raw material solution at 50 to 90°C under reduced pressure using an evaporator, followed by drying at 50 to 150°C for 1 to 48 hours employing a vacuum dryer; a method which involves drying the catalyst raw material solution by spraying on a hot plate heated at 150 to 300°C through a nozzle; and a method which involves drying with a spray drier (spray hot air drier). Industrially, the drying with the spray drier is preferable. The spray drier refers to a hot air drier consisting of a drying chamber, a raw material solution spraying portion, hot air intaking and exhausting portions, and a dry powder recovering portion. A preferred procedure of spray drying includes feed of the catalyst raw material solution by a pump and then spraying the solution into a drying chamber via a rotary atomizer (centrifugal atomizer), a pressure nozzle, a two fluid nozzle (gas atomizer), or the like. The droplet of the sprayed catalyst raw material solution is countercurrently or concurrently contacted with hot air controlled at an inlet temperature of 150 to 500°C to evaporate the solvent, and recovered in the form of a dried powder.

A method for calcining the dried catalyst precursor thus obtained is not particularly restricted. Preferably, the calcination is carried out in an electric furnace at 400 to 1,000°C for 0.5 to 48 hours under the flow of an inert gas such as nitrogen and/or an oxygen-containing gas. To improve the dispersibility of active species, treatment with steam may be further performed at 150 to 500°C for 0.5 to 48 hours before or after the calcination.

Since the reaction according to the method of the invention is carried out using a fluid bed reaction system as described below, a method is particularly preferable which involves drying the catalyst raw material solution using a spray drier to provide a shaped catalyst precursor, which is then calcined at 500 to 800°C for 1 to 24 hours with flowing an oxygen-containing gas.

The reaction according to the method of the invention for producing an alcohol and/or ketone will be then described.

The reaction according to the method of the invention refers to a reaction which involves contacting and reacting a raw material containing at least one alkene with an oxide catalyst in the presence of steam in a gas phase under a condition where molecular oxygen is not fed to produce an alcohol and/or a ketone corresponding to the alkene.

The mechanism of the reaction is uncertain, but the present inventors have deduced that the hydration reaction of alkene and steam generates an alcohol, which then causes an oxidative dehydrogenation reaction with the lattice oxygen of the oxide catalyst to produce a ketone.

Preferred examples of the alkene contained in the raw material include propylene, 1-butene, 2-butene (cis and/or trans), pentene, hexene, cyclohexene, heptene, octene, and cyclooctene. Propylene, 1-butene, 2-butene (cis and/or trans), and cyclohexene are more preferable; and 1-butene and 2-butene (cis and/or trans) are particularly preferable. These alkenes may be used singly or as a mixture thereof.

Any of gases inert to the reaction such as nitrogen, argon, carbon dioxide, methane, ethane, propane, or butane may be mixed or entrained as a diluent or carrier gas in the reaction raw material.

The molar ratio of the amount of steam fed to a reactor/the amount of alkene fed to the reactor is preferably 0.05 or more in terms of reaction rate and 10.0 or less in terms of effect, more preferably 0.2 to 5.0, particularly preferably 0.5 to 2.0.

Without feed of molecular oxygen to the reactor, the lattice oxygen of the oxide catalyst may be used as a main oxygen source for reaction.

The ratio of the amount of alkene fed to the amount of catalyst (weight hourly space velocity (WHSV)) is not particularly restricted. It is preferably 0.01 to 10 $Hr^{-1}$, more preferably 0.05 to 5 $Hr^{-1}$, particularly preferably 0.1 to 2 $Hr^{-1}$.

The weight hourly space velocity (WHSV) is defined by the following equation.

WHSV ($Hr^{-1}$) = the feed rate of the alkene (kg/Hr)/the amount of the catalyst (kg)

The preferable range of reaction temperature varies depending on the raw materials, but is typically 130 to 500°C, more preferably 200 to 450°C, particularly preferably 230 to 350°C. The reaction pressure is not particularly restricted, but preferably 0.01 to 5 MPa, more preferably 0.01 to 1 MPa, even more preferably 0.03 to 0.5 MPa, particularly preferably 0.05 to 0.3 MPa.

The reaction system used in the method of the invention is carried out by the use of a so-called catalyst circulation system wherein the following procedure is repeated: while carrying out the reaction by a fluid bed reaction system, the catalyst used for the reaction is continuously or intermittently withdrawn from a reactor to send into a regenerator for regeneration and the whole or part of the regenerated catalyst is continuously or intermittently returned to the fluid bed reactor. The amount of catalyst circulated is determined so as to make the conversion of reaction constant. The mass ratio of the amount of catalyst returned to the reactor/the amount of alkene fed to the reactor is preferably in the range of 0.5 to 100, more preferably 5 to 100, particularly preferably 5 to 70.

The catalyst is subjected to regeneration in an oxygen-containing atmosphere at a temperature and a residence time that are capable for the regeneration thereof. The regeneration temperature is preferably in the range of 100 to 550°C, more preferably 270 to 550°C, particularly preferably 270 to 500°C. The regeneration time is preferably in the range of 1 second to 10 hours, more preferably 10 seconds to 10 hours, particularly preferably 1 minute to 1 hour. The oxygen gas concentration is preferably in the range of 10 volume ppm to 100 volume%, more preferably 10 volume ppm to 21 volume%. In the regeneration of catalyst, it is preferable to select, from combinations of the above-described ranges, conditions of reoxidizing the catalyst reduced by the reaction to recover the activity thereof as well as not completely removing carbonaceous substances accumulated on the catalyst and intentionally leaving certain amounts of carbonaceous substances on the catalyst to inhibit the further generation of the carbonaceous substances to improve selectivity for the objective substance.

According to the invention, oxygen gas introduced into the reactor from the regenerator can be decreased by providing a stripper on the way from the regenerator to the reactor to strip the catalyst which the above-described inert gas carries from the regenerator to the reactor. An inert gas such as $N_2$, carbon dioxide gas, or steam is passed through the stripper and countercurrently or concurrently contacted with the catalyst carried from the regenerator to the reactor (this operation is defined as "stripping" and the location at which the operation occurs, as "stripper"). It has a highly beneficial effect in maintaining the selectivity of the objective product to provide the stripper on the way from the regenerator to the reactor because the contamination of oxygen gas in the reactor increases by-products due to excessive oxidation and reduces the selectivity of the objective product.

A stripper may be also provided on the way from the reactor to the regenerator. Providing the stripper in this place can decrease the raw material alkene which is adsorbed to, or entrained by, the catalyst, carried to the regenerator, and lost due to combustion or by disposal.

Fig. 1 shows a schematic diagram consisting of a fluid bed reactor, a regenerator, and strippers (in the case where they are provided on the way from the regenerator to the reactor and on the way from the reactor to the regenerator).

The arrangement and shape of the stripper are not particularly restricted. It may be integral with the reactor vessel or regenerator vessel, or may be provided in separate vessel therefrom. In the case of integration, it is preferable to provide a stripping zone in a lower extended portion of the reactor vessel or regenerator vessel, to which the above-described inert gas is fed for stripping.

Conditions for stripping are not particularly restricted. The ratio of the volume of inert gas fed/the mass of catalyst carried (volume/mass ratio: l/kg) is preferably 0.1 to 1,000, more preferably 1 to 500, particularly preferably 1 to 200. The stripping temperature is preferably 0 to 500°C, more preferably 0 to 300°C, particularly preferably 5 to 200°C. The stripping time is preferably 0.1 second to 10 Hrs, more preferably 1 second to 5 Hrs, particularly preferably 30 seconds to 1 Hr. Preferably, the flow of the catalyst is countercurrently contacted with the flow of the inert gas.

From a reaction mixture containing an alcohol and/or a ketone which is obtained by the reaction as described above, the alcohol and/or the ketone may be recovered through a known recovering, separating, and purifying operation such as cooling, distillation or extraction. After separation from the reaction mixture, unreacted alkene may be optionally recycled for using as part of the reaction raw material.

By way of example, when MEK is produced from 1-butene and/or 2-butene, the reaction mixture is cooled to condense MEK and steam. This is subjected to gas/liquid separation, followed by recovering MEK from the condensate. After recovering MEK, the whole or part of the recovered water containing by-products such as acetic acid is again recycled to be fed to the reactor in the form of steam. An uncondensed gas phase is subjected to the liquefying and recovering of MEK entrained by the gas phase through compression and cooling, and unreacted 1-butene and/or 2-butene are optionally subjected to the separation of light gases such as carbon dioxide and again recycled wholly or partially to be fed to the reactor.

<Examples>

The invention is described below in further detail, based on Examples and Comparative Examples. However, this invention is not intended to be limited to these examples. The analysis equipment and analysis conditions used in Examples and Comparative Examples will be first described.

(Reaction gas analysis)

Gas chromatograph: Shimadzu GC-17A, capillary column: SPB-1 ($\phi$0.25 mmx60 m), injector temperature: 250°C, FID temperature: 250°C, column temperature: 40°C $\times$ 10 min., 5°C/min. to 200°C, 200°C $\times$ 8 min. held.

(Analysis of carbon dioxide and carbon monoxide in the reaction gas)

Gas chromatograph: Shimadzu GC-8A, packed column: a parallel column of Porapac Q ($\phi$3 $\times$ 2 m) and MS-5A ($\phi$3 $\times$ 3 m), injector temperature: 70°C, TCD temperature: 70°C, column temperature: 70°C held.

(Analysis of chemical composition of catalyst)

EPMA (Scanning Electron Microanalyzer) X-650 from Hitachi Ltd.

[Reference Example 1] (Preparation of catalyst A)

In 60 L of pure water was dissolved 9,380 g of stannic chloride pentahydrate, to which 3,040 g of silica fine powder (Aerosil 200V (trade name) from Nippon Aerosil Co., Ltd.) was then added with stirring at 500 rpm, followed by adding 8 mass% aqueous ammonia until the pH reaches 5 to 7 to provide a white precipitate consisting of silica and tin hydroxide. The white precipitate was filtered and then well washed with pure water. To this cake was added 660 g of ammonium molybdate dissolved in 12.7 L of pure water to make a homogeneous slurry, to which concentrated nitric acid was then added so as to provide a slurry pH of 2 to 4. This slurry was spray dried using a spray drier to provide a spherical molding powder. The molding powder obtained was calcinated at 650°C for one hour under an atmosphere of air in an electric furnace. When the composition of this catalyst A was analyzed using the EPMA composition analyzer, it was shown to consist of 51 mass% of $SnO_2$, 7 mass% of $MoO_3$, and 42 mass% of $SiO_2$. Catalyst A had an Mo/(Sn + Mo) atomic ratio of 0.13, was in the form of a smooth sphere suitable for a fluid bed catalyst, and had sufficient mechanical strength.

[Reference Example 2] (Preparation of catalyst B)

Catalyst B having a different composition was prepared in about the same way as that in Reference Example 1. The composition of catalyst B consisted of 48 mass% of $SnO_2$ 11 mass% of $MoO_3$, and 41 mass% of $SiO_2$. Catalyst B had an Mo/(Sn + Mo) atomic ratio of 0.19, was in the form of a smooth sphere suitable for a fluid bed catalyst, and had sufficient mechanical strength.

[Reference Example 3] (Preparation of catalyst C)

Catalyst C having a different composition was prepared in about the same way as that in Reference Example 1. The composition of catalyst C consisted of 65 mass% of $SnO_2$, 5 mass% of $MoO_3$, and 30 mass% of $SiO_2$. Catalyst C had an Mo/(Sn + Mo) atomic ratio of 0.07, was in the form of a smooth sphere suitable for a fluid bed catalyst, and had sufficient mechanical strength. [Reference Example 4] (Preparation of catalyst D)

Catalyst D was prepared in about the same way as that in Reference Example 1. The composition of catalyst D consisted of 31 mass% of $SnO_2$, 30 mass% of $MoO_3$, and 39 mass% of $SiO_2$. Catalyst D had an Mo/(Sn + Mo) atomic ratio of 0.50, and was unfavorable for a fluid bed catalyst because the molding powder thereof aggregated with each other and could not be uniformly calcinated.

This shows that Mo/(Sn + Mo) is preferably less than 0.50 for a fluid bed catalyst.

[Reference Example 5] (Preparation of catalyst E)

Catalyst E comprising oxides of Ti and Mo was prepared in substantially the same way as that in Reference Example 1 except for the use of titanium tetrachloride in place of stannic chloride pentahydrate. The composition of catalyst F consisted of 44 mass% of $TiO_2$, 17 mass% of $MoO_3$, and 39 mass% of $SiO_2$. Catalyst F had an Mo/(Ti + Mo) atomic ratio of 0.18, was in the form of a smooth sphere suitable for a fluid bed catalyst, and had sufficient mechanical strength.

[Reference Example 6] (Preparation of catalyst F)

Catalyst F having a different composition was prepared in substantially the same way as that in Reference Example 1. The composition of catalyst F consisted of 46 mass% of $SnO_2$, 16 mass% of $MoO_3$, and 38 mass% of $SiO_2$. Catalyst B had an Mo/(Sn + Mo) atomic ratio of 0.29, was in the form of a smooth sphere suitable for a fluid bed catalyst, and had sufficient mechanical strength.

[Example 1]

Catalyst A was packed into a reaction apparatus comprising a fluid bed reactor and a catalyst regenerator as shown in Fig. 1, followed by carrying out a fluid bed reaction using a catalyst circulation system wherein the reaction and catalyst regeneration were continuously conducted while circulating catalyst A between the reactor and regenerator. At this time, a stripper device (internal diameter $\phi$20 × length 60) was provided between the regenerator and the reactor; $N_2$ was fed at a ratio of the volume of inert gas fed/the mass of catalyst carried (volume/mass ratio: l/kg) of 67; and the regenerated catalyst before returning to the reactor was stripped at 150°C. A raw material having a ratio of 1-butene/steam/$N_2$ of 20/50/30 (volume ratio) was fed to the reactor at a weight hourly space velocity (WHSV) of 0.2 $Hr^{-1}$ based on the amount of catalyst in the reactor. The amount of 1-butene fed was 25.6 Nl/Hr. The reaction temperature was 250°C. A mixed gas of air and $N_2$ was fed to the regenerator. The reaction was conducted for 10 hours. The reaction results at 1 Hr and 3 Hrs are shown in Table 1. For by-products, the selectivity of $CO_x$ (the total of $CO_2$ and CO) is given as a representative therefor.

Each of the properties shown in Table 1 is defined as follows. All are based on the amount of carbon.

$$\text{The conversion (\%) of 1-butene} = (F-L)/F \times 100$$

$$\text{The selectivity (mol\%) of each component} = P/(F-L) \times 100$$

F: the amount (Cmol) of 1-butene fed

L: the amount (Cmol) of unreacted 1-butene (including the amount of 2-butene generated by the isomerization of 1-butene)

P: the amount (Cmol) of each component produced

2-Butene, the isomerization product of 1-butene, was considered as an unreacted material because it can be reused as a raw material.

By-products produced in addition to MEK refer to, for example, $CO_2$ CO, acetone, acetic acid, butyl alcohol, and oligomers having 5 or more carbon atoms. [Comparative Example 1]

A fluid bed reaction was carried out using a catalyst circulation system in about the same conditions as those in Example 1 except for directly returning the catalyst from the regenerator to the reactor without stripping. The reaction was conducted for 10 hours. The reaction results at 1 Hr and 3 Hrs are shown in Table 1. For by-products, the selectivity of $CO_x$ (the total of $CO_2$ and CO) is given as a representative therefor.

[Example 2]

Except for supplying at a weight hourly space velocity (WHSV) of 0.4 $Hr^{-1}$, stripping was performed in substantially the same way as that in Example 1 and a fluid bed reaction was carried out using a catalyst circulation system under substantially the same conditions. The reaction was conducted for 10 hours. The reaction results at 1 Hr and 3 Hrs are shown in Table 1. For by-products, the selectivity of $CO_x$ (the total of $CO_2$ and CO) is given as a representative therefor.

[Comparative Example 2]

A fluid bed reaction was carried out using a catalyst circulation system under substantially the same conditions as those in Example 2 except for directly returning the catalyst from the regenerator to the reactor without stripping. The reaction was conducted for 10 hours. The reaction results at 1 Hr and 3 Hrs are shown in Table 1. For by-products, the selectivity of $CO_x$ (the total of $CO_2$ and CO) is given as a representative therefor.

Comparisons between Example 1 and Comparative Example 1 and between Example 2 and Comparative Example 2 indicate that stripping may be carried out on the way from the regenerator to the reactor to improve the selectivity of the objective product.

[Example 3]

A fluid bed reaction was conducted using a catalyst circulation system under substantially the same conditions as those in Example 1 except for the use of catalyst B. The reaction was conducted for 10 hours. The reaction results at 1 Hr and 3 Hrs are shown in Table 1. For by-products, the selectivity of $CO_x$ (the total of $CO_2$ and CO) is given as a representative therefor.

[Example 4]

A fluid bed reaction was conducted using a catalyst circulation system under substantially the same conditions as those in Example 1 except for the use of catalyst C. The reaction was conducted for 10 hours. The reaction results at 1 Hr and 3 Hrs are shown in Table 1. For by-products, the selectivity of $CO_x$ (the total of $CO_2$ and CO) is given as a representative therefor.

[Example 5]

A fluid bed reaction was conducted using a catalyst circulation system under substantially the same conditions as those in Example 1 except for the use of catalyst E. The reaction was conducted for 10 hours. The reaction results at 1 Hr and 3 Hrs are shown in Table 1. For by-products, the selectivity of $CO_x$ (the total of $CO_2$ and CO) is given as a representative therefor.

[Example 6]

A fluid bed reaction was conducted using a catalyst circulation system under substantially the same conditions as those in Example 1 except for the use of catalyst F. The reaction was conducted for 10 hours. The reaction results at 1 Hr and 3 Hrs are shown in Table 1. For by-products, the selectivity of $CO_x$ (the total of $CO_2$ and CO) is given as a representative therefor. The catalyst used in this Example had a slightly increased content of molybdenum (within the range defined in the invention) compared to that of each catalyst used in other Examples; in this case, the selectivity of the objective substance MEK was still excellent, but slightly decreased compared to that in each of other Examples.

In addition, it was noted that a reaction similar to that in each Example, in which catalyst A was used and the raw material alkene was changed from 1-butene to propylene or cyclohexene, could improve the selectivity of the objective product when a stripping section was provided between the regenerator and the reactor to strip the catalyst being returned from the regenerator to the reactor.

Table 1

| | Catalyst | Catalyst composition Mo/(Sn + Mo) (atomic ratio) | WHSV ($HR^{-1}$) | Stripping on the way from a regenerator to a reactor | Reaction time | Conversion (%) | MEK selectivity (%) | $CO_x$ selectivity |
|---|---|---|---|---|---|---|---|---|
| Example 1 | A | 0.13 | 0.2 | yes | 1 Hr | 14.9 | 94.0 | 0.8 |
| | | | | | 3 Hr | 16.0 | 94.5 | 0.4 |
| Comparative Example 1 | A | 0.13 | 0.2 | no | 1 Hr | 11.8 | 89.0 | 2.2 |
| | | | | | 3 Hr | 11.3 | 88.8 | 2.6 |
| Example 2 | A | 0.13 | 0.4 | yes | 1 Hr | 10.0 | 97.2 | 0.2 |
| | | | | | 3 Hr | 9.0 | 93.2 | 0.3 |
| Comparative Example 2 | A | 0.13 | 0.4 | no | 1 Hr | 8.9 | 84.9 | 2.4 |
| | | | | | 3 Hr | 8.8 | 80.3 | 2.0 |
| Example 3 | B | 0.19 | 0.2 | yes | 3 Hr | 16.0 | 94.0 | 0.3 |
| Example 4 | C | 0.07 | 0.2 | yes | 3 Hr | 13.5 | 94.0 | 0.1 |
| Example 5 | E | 0.18[*1] | 0.2 | yes | 3 Hr | 16.0 | 93.5[*2] | 0.3 |
| Example 6 | F | 0.29 | 0.2 | yes | 3 Hr | 14.0 | 90.5 | 0.8 |

*1: This indicates Mo/(Ti + Mo) (atomic ratio).
*2: This indicates the total of selectivities of MEK and butanol only in Example 5. The ratio of the selectivity of MEK to that of butanol was about 1 : 1.

INDUSTRIAL APPLICABILITY

The production method of the invention has advantages that the selectivity of the objective product is improved when the reaction for producing, from at least one kind of alkene, a corresponding alcohol and/or ketone using an oxide catalyst in the presence of steam in a gas phase is carried out using a system wherein the catalyst is circulated between a reactor and a regenerator. Thus, it is useful as an industrial method for producing these compounds.

**Claims**

1. A method for producing an alcohol and/or a ketone, wherein a raw material containing at least one alkene is contacted and reacted with an oxide catalyst in the presence of steam in a gas phase to produce an alcohol and/or a ketone corresponding to said alkene(s), which comprises satisfying the following requirements of (a) to (c):

   (a) said oxide catalyst contains an oxide(s) of molybdenum and/or tin;
   (b) said reaction is carried out under a condition where molecular oxygen is not fed and by the use of a system wherein said catalyst is circulated between a fluid bed reactor and a regenerator; and
   (c) a stripper is provided on the way from said regenerator to said reactor.

2. The method according to claim 1, wherein a stripper is further provided on the way from said reactor to said regenerator.

3. The method according to claim 1 or 2, wherein said alkene(s) is 1-butene and/or 2-butene.

4. The method according to any one of claims 1 to 3, wherein the atomic ratio X of molybdenum to the sum of tin and molybdenum contained in said oxide catalyst ((Mo/(Sn + Mo) where Mo is the number of molybdenum atoms in said oxide catalyst and Sn is the number of tin atoms in said oxide catalyst) is in the range of $0 \leq X < 0.50$.

5. The method according to any one of claims 1 to 3, wherein the atomic ratio X of molybdenum to the sum of tin and molybdenum contained in said oxide catalyst ((Mo/(Sn + Mo) where Mo is the number of molybdenum atoms in said oxide catalyst and Sn is the number of tin atoms in said oxide catalyst) is in the range of $0.01 \leq X \leq 0.24$.

# FIG.1

REACTION GAS

REGENERATION GAS

FLUID BED REACTION ZONE

CATALYST REGENERATOR

b

a

RAW MATERIAL GAS

OXYGEN-CONTAINING GAS

STRIPPING ZONE

STRIPPER

INERT GAS

INERT GAS

INERT GAS

## INTERNATIONAL SEARCH REPORT

| | International application No. |
|---|---|
| | PCT/JP2005/001842 |

A. CLASSIFICATION OF SUBJECT MATTER
Int.Cl⁷ C07C27/00, 29/04, 31/12, 45/27, 49/10, C07B61/00

According to International Patent Classification (IPC) or to both national classification and IPC

B. FIELDS SEARCHED

Minimum documentation searched (classification system followed by classification symbols)
Int.Cl⁷ C07C27/00, 29/00, 31/00, 45/00, 49/00

Documentation searched other than minimum documentation to the extent that such documents are included in the fields searched

Electronic data base consulted during the international search (name of data base and, where practicable, search terms used)
CAPLUS(STN)

C. DOCUMENTS CONSIDERED TO BE RELEVANT

| Category* | Citation of document, with indication, where appropriate, of the relevant passages | Relevant to claim No. |
|---|---|---|
| A | JP 49-61112 A (Stamicarbon B.V.), 13 June, 1974 (13.06.74), Pages 1 to 2 & GB 1435784 A & US 3987104 A | 1-5 |
| A | JP 47-8046 B (STAMICARBON N.V.), 08 March, 1972 (08.03.72), Pages 1 to 2 & GB 1240858 A | 1-5 |
| A | JP 49-34652 B (STAMICARBON N.V.), 17 September, 1974 (17.09.74), Pages 1 to 2 & GB 1324717 A | 1-5 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 06 April, 2005 (06.04.05) | 26 April, 2005 (26.04.05) |

| Name and mailing address of the ISA/ | Authorized officer |
|---|---|
| Japanese Patent Office | |
| Facsimile No. | Telephone No. |

Form PCT/ISA/210 (second sheet) (January 2004)

**INTERNATIONAL SEARCH REPORT**

International application No.

PCT/JP2005/001842

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2002-513845 A  (Exxon Research & Engineering Co.),<br>14 May, 2002 (14.05.02),<br>Pages 6 to 11<br>& WO 99/57225 A1          & US 6093867 A<br>& EP 1112336 A1 | 1-5 |
| A | JP 8-501019 A  (Exxon Research & Engineering Co.),<br>06 February, 1996 (06.02.96),<br>Pages 8 to 13, 21 to 24<br>& WO 94/5418 A1          & US 5756414 A<br>& EP 671978 A1 | 1-5 |

Form PCT/ISA/210 (continuation of second sheet) (January 2004)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 49072209 A
- JP 59163335 A
- JP 47008046 B
- JP 49061112 A
- JP 49034652 B

**Non-patent literature cited in the description**

- Kagaku Binran Kisohen. Maruzen Co., Ltd, 1993, vol. I, 1-56